# EUROPEAN PATENT APPLICATION

(11) **EP 1 961 435 A2**
(43) Date of publication of application: **27.08.2008**
(21) Application number: 08250598.3
(22) Date of filing: 20.02.2008
(51) Int. Cl.: A61L 31/16

(54) **Stent and method for reducing tissue damage after ischemic injury with thymosin B4**

(30) Priority: 21.02.2007 US 891003 P
(71) Applicant: Innovational Holdings, LLC, New Brunswick, NJ 08933 (US)
(72) Inventor: Forrester, James, Malibu, CA 90265 (US); Parker, Theodore L., Danville, CA 94506 (US); Luk, Andrew Sheung-King, Catro Valley, CA 94552 (US)
(74) Representative: Warner, James Alexander

(57) **Abstract**

Methods and devices are provided for the delivery of thymosin β4 or an isoform thereof which reduces myocardial tissue damage due to ischemia. The thymosin β4 or isoform is delivered to the myocardial tissue over an administration period sufficient to achieve reduction in ischemic or reperfusion injury of the myocardial tissue. In addition to the treatment of ischemic injury, the thymosin β4, its analogues or derivatives can also be delivered from a stent to treat heart muscle loss from other causes including myocarditis, chronic heart failure, or prior AMI.

## Description

### FIELD OF THE INVENTION

This invention is directed to a stent and method for the local delivery of thymosin β4, analogs and derivatives thereof for reduction in tissue damage due to ischemia or otherwise improving heart muscle function. More particularly, this invention relates to the local delivery of therapeutic agents from implantable medical devices to reduce myocardial tissue damage after ischemic injury, chronic heart failure, or other heart damage.

### BACKGROUND OF THE INVENTION

The reduction or cessation of blood flow to a vascular bed ("ischemia") accounts for a variety of clinical events that require immediate intervention and restitution of adequate perfusion to the jeopardized organ or tissue. Different tissues can withstand differing degrees of ischemic injury. However, tissues may progress to irreversible injury and cellular necrosis if not reperfused.

Impaired perfusion of cardiac tissue results in a loss of the heart's ability to function properly as the tissue becomes oxygen and energy deprived. Permanent injury is directly related to the duration of the oxygen deficit the myocardium experiences. Ischemia occurs when blood flow to an area of cells is insufficient to support normal metabolic activity. Surgical and percutaneous revascularization techniques following acute myocardial infarction (AMI) are highly effective for treating ischemic myocardial tissue. In the case of an AMI, the main blood flow is stopped by the blockage of a coronary artery and the tissue is perfused only through collateral arteries. Reperfusion is the term used to describe the act of reestablishing blood flow and oxygen supply to ischemic tissue. Reperfusion is essential to the future survival of cells within an ischemic area. Reperfusion may be achieved by a blood flow recanalization therapy, such as coronary angioplasty, administration of a thrombolytic drug, or coronary artery bypass surgery. Timely reperfusion of ischemic myocardium limits infarct size. Early reperfusion with angioplasty or thrombolytic therapy reduces myocardial damage, improves ventricular function, and reduces mortality in patients with AMI. Myocardial salvage can be compromised by such complications as coronary reocclusion and severe residual coronary stenosis.

Reperfusion of the ischemic myocardium does not alone return full functioning of the myocardium. In fact, it is well known that reperfusion itself can cause damage to many cells that survive the initial ischemic event. Studies have shown that reperfusion may accelerate death of irreversibly injured myocardium, and may also compromise survival of jeopardized, but still viable, myocytes salvaged by reperfusion. These so-called reperfusion injuries may represent more than 50% of the ultimate infarct size. A number of mechanisms, including apoptosis, inflammation, collagen expression, and remodeling, are believed to be responsible for ischemia-induced reperfusion injury. Development of adjuvant treatments to protect the post-ischemic myocardium and maximize benefits of coronary reperfusion has therefore become a major target of modem cardiovascular research.

Compounds capable of minimizing and containing ischemic or reperfusion damage represent important therapeutic agents. In past years, it has been demonstrated that mortality rates following myocardial infarction and reperfusion can be further reduced by delivery of drugs which optimize energy transfer in post-ischemic heart tissue.

In general, the compounds which have been used for reducing tissue damage after acute myocardial infarction have been delivered systemically, such as intravenously or by arterial infusion. Systemic delivery of these compounds have significant drawbacks including the requirement for additional administration of protective agents to prevent damage to non-target tissues caused by systemic delivery, i.e. requirement for delivery of glucose and potassium with an insulin infusion. Other drawbacks include the requirement for continuous administration and supervision, suboptimal delivery to the ischemic area, patient discomfort, high dosages required for systemic delivery, and side effects of the systemic delivery from high drug dosages.

To overcome such problems, local delivery of therapeutic agents for reducing ischemia-induced tissue damage, such as insulin, from a stent or catheter has been described in U.S. Patent Application Publication No. 2004/0142014. Local delivery of therapeutic agents provides the advantage of reduction of ischemic injury, including reduction of reperfusion injury, without the difficulties associated with systemic delivery of high doses of the therapeutic agent. U.S. Patent Application Publication No. 2004/0142014 also describes incorporating antirestenotic agents to inhibit restenosis following stent implantation.

It is an object of the invention to provide methods and devices to reduce tissue damage due to ischemic injury and restenosis by the local administration of anti-ischemic agents including thymosin β4, its analogues, and derivatives.

### BRIEF SUMMARY OF THE INVENTION

Methods and devices are provided for the delivery of anti-ischemic agents including thymosin β4, its analogues and derivatives which reduce myocardial tissue damage due to ischemia. The anti-ischemic agents are delivered to the myocardial tissue over an administration period sufficient to achieve reduction in ischemic or reperfusion injury of the myocardial tissue. In addition to the treatment of ischemic injury, the thymosin β4 or its analogues or derivatives can also be delivered from a stent to treat heart muscle loss from other causes including myocarditis, chronic heart failure, or prior AMI.

In a preferred embodiment, the therapeutic agents are delivered using an implanted or insertable device releasing an effective amount of the anti-ischemic agent and one or more anti-restenotic agents.

### BRIEF DESCRIPTION OF THE DRAWINGS

Figure 1 is a cross-sectional perspective view of a portion of an expandable medical device implanted in the lumen of an artery with a therapeutic agent arranged for delivery to the lumen of the artery.

Figure 2 is a perspective view of an expandable medical device showing a plurality of openings.

Figure 3 is an enlarged side cross-sectional view of a portion of the expandable medical device of Figure 2.

Figure 4 is an enlarged side cross-sectional view of an opening illustrating a first therapeutic agent provided for delivery to a lumen of the blood vessel and a second therapeutic agent provided for delivery to a wall of the blood vessel.

Figure 5 is an enlarged side cross-sectional view of an opening illustrating first and second therapeutic agents for delivery to a lumen of the blood vessel.

### DETAILED DESCRIPTION OF THE INVENTION

Stents and methods are provided for treatment of acute ischemic syndromes including acute myocardial infarction and for reducing injury due to reperfusion of tissue with thymosin β4, its analogues, isoforms, and derivatives.

### I. Definitions

First, the following terms, as used herein, shall have the following meanings:

The terms "drug" and "therapeutic agent" are used interchangeably to refer to any therapeutic, prophylactic or diagnostic agent.

The term "anti-ischemic agent" is used to refer to a drug or therapeutic agent that reduces tissue damage due to ischemia and/or reperfusion, or reduces infarct size after AMI.

The term "matrix" refers to a material that can be used to contain or encapsulate a therapeutic, prophylactic or diagnostic agent. As described in more detail below, the matrix may be polymeric, natural or synthetic, hydrophobic, hydrophilic or lipophilic, bioresorbable or non-bioresorbable. The matrix will typically be biocompatible. The matrix typically does not provide any therapeutic responses itself, though the matrix may contain or surround a therapeutic agent, and/or modulate the release of the therapeutic agent into the body. A matrix may also provide support, structural integrity or structural barriers.

The term "biocompatible" refers to a material that, upon implantation in a subject, does not elicit a detrimental response sufficient to result in the rejection of the matrix.

The term "bioresorbable" refers to a matrix, as defined herein, that can be broken down by either a chemical or physical process, upon interaction with a physiological environment, typically into components that are metabolizable or excretable, over a period of time from minutes to years, preferably less than one year.

The term "ischemia" refers to a lack of oxygen in a region or tissue. The term typically refers to local hypoxia resulting from obstructed blood flow to an affected tissue.

The term "ischemic injury" as used herein refers to both injury due to obstructed blood flow and reperfusion injury caused by removal of the obstruction and restoration of blood flow.

The term "openings" includes both through openings and recesses.

The term "pharmaceutically acceptable" refers to the characteristic of being non-toxic to a host or patient and suitable for maintaining the stability of a beneficial agent and allowing the delivery of the beneficial agent to target cells or tissue.

The term "polymer" refers to molecules formed from the chemical union of two or more repeating units, called monomers. The term "copolymer" refers to molecules joined from the chemical union of two or more different monomers. The term "polymer" includes dimers, trimers and oligomers. The polymer may be synthetic, naturally-occurring or semisynthetic. In a preferred form, the term "polymer" refers to molecules which typically have a M_{w} greater than about 3000 and preferably greater than about 10,000 and a M_{w} that is less than about 10 million, preferably less than about a million and more preferably less than about 200,000. Examples of polymers include, but are not limited to, poly-alpha-hydroxy acid esters such as polylactic acid (PLA or DLPLA), polyglycolic acid, polylactic-co-glycolic acid (PLGA), polylactic acid-co-polycaprolactone (PLA/PCL); poly (block-ethylene oxide-block-lactide-co-glycolide) polymers such as (PEO-block-PLGA and PEO-block-PLGA-block-PEO); polyethylene glycol and polyethylene oxide, poly (block-ethylene oxide-block-propylene oxide-block-ethylene oxide); polyvinyl pyrrolidone (PVP); polyorthoesters; polysaccharides and polysaccharide derivatives such as polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclo dextrin sulfo butyl ethers; polypeptides and proteins such as polylysine, polyglutamic acid, and albumin; polyanhydrides; polyhydroxy alkanoates such as polyhydroxy valerate and polyhydroxy butyrate.

The term "restenosis" refers to the re-narrowing of an artery following a cardiac procedure such as angioplasty which may include stenosis following stent implantation.

The term "anti-restenotic agent" refers to a compound that can reduce or prevent restenosis as described above.

The term "apoptosis" refers to a form of cell death in which a programmed series of events leads to elimination of cells.

### II. Drug Delivery Devices

Local drug delivery devices, for example, devices in the form of catheters, polymeric delivery devices, and/or stents, can be used to deliver therapeutic agents to ischemic areas, such as myocardial tissue at and downstream of the implantation site when positioned directly at or near a site of a previously occluded blood vessel. The delivery of an anti-ischemic agent locally at the ischemic injury site improves the viability of the cells by reducing ischemic injury to the myocardial cells including reperfusion injury which may occur upon return of blood flow to the ischemic tissue. In cases where reperfusion therapy is performed by angioplasty, a stent is often delivered to the reopened occlusion site. A drug delivery stent for delivery of a therapeutic agent for treatment of ischemic injury and/or anti-restenotic agent can be implanted at the implantation site in the traditional manner after angioplasty. The drug delivery stent for delivery of the therapeutic agent implanted at or near the occlusion site following reperfusion therapy provides the advantage of reduction of ischemic injury including reduction of reperfusion injury without the difficulties associated with systemic delivery of the therapeutic agent. The implantable medical device may also contain one or more drugs that sensitize tissue to the anti-ischemic agent.

Delivery devices can consist of something as simple as a catheter which delivers drug into a blood vessel for release downstream to the affected tissue; injection catheters or devices for injection of drug directly into the myocardium, polymeric devices which can be in the form of coatings; pellets; particles which contain bioactive molecules that are released by diffusion or degradation of the polymer over time; or a stent. The advantage of the stent is that it can serve the dual purpose of a scaffolding within the blood vessel and release of the bioactive molecules.

Examples of devices for administration of biologically active agent include artificial organs, anatomical reconstruction prostheses, permanent and biodegradable stents, including peripheral stents and coronary stents, vascular grafts and conduits vascular shunts, biological conduits, valve grafts, permanently in-dwelling percutaneous devices, and combinations thereof. Other biomedical devices that are designed to dwell for extended periods of time within a patient that are suitable for the inclusion of therapeutic agents include, for example, Hickman catheters and other percutaneous articles that are designed for use over a plurality of days. Polymeric delivery devices include, for example, U.S. Patent Nos. 6,491,617 to Ogle, et al., 5,843,156, and 6,290,729 to Slepian, et al. In Slepian, et al., the therapeutic agent is incorporated into a polymeric material which is applied as a thermoplastic coating that is heated to conform to the surface of a vessel, or more preferably, applied in a polymeric material that is in a fluent state at the time of application and photopolymerized in situ.

Examples of methods and materials for application and release of therapeutic agents in a polymeric coating on an implantable medical device are described in U.S. Patent Nos. 6,273,913 to Wright, et al. and 6,712,845 to Hossainy.

One approach has been to coat a medical device such as a vascular stent with a biologically active agent contained in a polymer matrix, the device may be directly coated with a biologically active agent without a polymer matrix. The compound can be attached using any means that provide a drug-releasing platform. Coating methods include, but are not limited to, dipping, spraying, precipitation, coacervation, vapor deposition, ion beam implantation, and crystallization. The biologically active agent when bound without a polymer can be bound covalently, ionically, or through other molecular interactions including, without limitation, hydrogen bonding and van der Waals forces.

Typically, a coating solution is applied to the device by either spraying a polymer solution onto the medical device or immersing the medical device in a polymer solution. Spraying in a fine spray such as that available from an airbrush will provide a coating with uniformity and will provide control over the amount of coating material to be applied to the medical device. With either a coating applied by spraying or by immersion, multiple application steps can be used to provide improved coating uniformity and improved control. The total thickness of the polymeric coating can range from about 0.1 micron to about 100 microns, preferably between about 1 micron and about 20 microns. The coating may be applied in one coat or, preferably, in multiple coats, allowing each coat to substantially dry before applying the next coat. In one embodiment the biologically active agent is contained within a base coat, and a top coat containing only polymer is applied over the biologically active agent-containing base coat to control release of the biologically active agent into the tissue and to protect the base coat during handling and deployment of the device.

As an alternative to coating an implantable medical device, the therapeutic agent can be deposited within holes, recesses or other macroscopic features within the implantable medical device. Methods for depositing a therapeutic agent into holes are described in U.S. Patent Publication No. 2004/0073294 which is incorporated herein by reference in its entirety.

The polymer can be a polymer that is biocompatible and should minimize irritation to the vessel wall when the medical device is implanted. For a stent coating, the polymer should also exhibit high elasticity/ductility, resistance to erosion, and controlled drug release. The polymer may be either a biostable or a bioresorbable polymer depending on the desired rate of release or the desired degree of polymer stability. Bioresorbable polymers that could be used for a coating or within openings include poly(L-lactic acid), polycaprolactone, poly(lactide-co-glycolide), poly(hydroxybutyrate-co-valerate), polydioxanone, polyorthoester, polyanhydride, poly(glycolic acid), poly(D,L-lactic acid), poly(glycolic acid-co-trimethylene carbonate), polyphosphoester, polyphosphoester urethane, poly(amino acids), cyanoacrylates, poly(trimethylene carbonate), poly(iminocarbonate), copoly(ether-esters) (e.g. PFO/PLA), polyalkylene oxalates, polyphosphazenes and biomolecules such as fibrin, fibrinogen, cellulose, starch, collagen and hyaluronic acid. Biostable polymers with a relatively low chronic tissue response such as polyurethanes, silicones, and polyesters could be used and other polymers could also be used if they can be dissolved and cured or polymerized on the medical device such as polyolefins, polyisobutylene and ethylene-alphaolefin copolymers; acrylic polymers and copolymers, ethylene-co-vinylacetate, polybutylmethacrylate, vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile, polyvinyl ketones; polyvinyl aromatics, such as polystyrene, polyvinyl esters, such as polyvinyl acetate; copolymcrs of vinyl monomers with each other and olefins, such as ethylene-methyl methacrylate copolymers, acrylonitrile-styrene copolymers, ABS resins, and ethylene-vinyl acetate copolymers (PEVA); polyamides, such as Nylon® 66 and polycaprolactam; alkyd resins; polycarbonates; polyoxymethylenes; polyimides; polyethers; epoxy resins, polyurethanes; rayon; rayon-triacetate; cellulose, cellulose acetate, cellulose butyrate; cellulose acetate butyrate; cellophane; cellulose nitrate; cellulose propionate; cellulose ethers; and carboxymethyl cellulose.

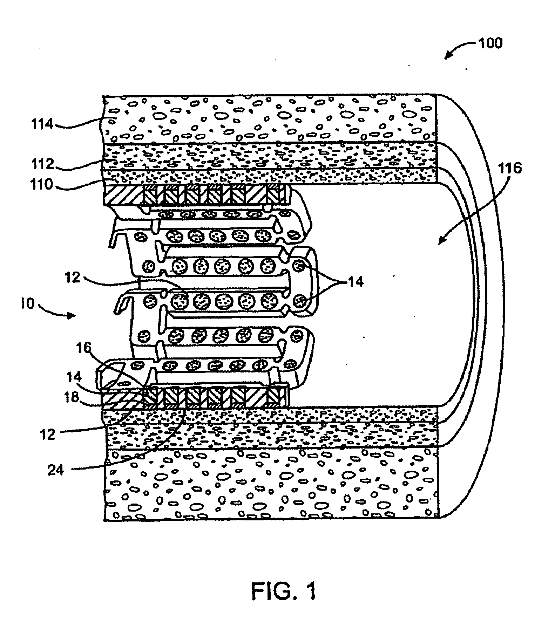

In a preferred embodiment, the device is an expandable stent including polymeric drug delivery reservoirs. Figure 1 illustrates an expandable medical device 10 in the form of a stent implanted in a lumen 116 of an artery 100. A wall of the artery 100 includes three distinct tissue layers, the intima 110, the media 112, and the adventitia 114. When the expandable medical device 10 is implanted in an artery at an occlusion site, one or more therapeutic agents delivered from the expandable medical device to the lumen 116 of the artery 100 are distributed locally to the tissue at the site of the occlusion and downstream by the blood flow.

One example of an expandable medical device 10, as shown in Figures 1-2, includes large, non-deforming struts 12, which can contain openings 14 without compromising the mechanical properties of the struts, or the device as a whole. The non-deforming struts 12 may be achieved by the use of ductile hinges 20 which are described in detail in U.S. Pat. No. 6,241,762. The openings 14 serve as large, protected reservoirs for delivering various therapeutic agents to the device implantation site and/or downstream of the implantation site.

The relatively large, protected openings 14, as described above, make the expandable medical device particularly suitable for delivering large amounts of therapeutic agents, or genetic or cellular agents, and for directional delivery of agents. The large non-defonning openings 14 in the expandable device 10 form protected areas or reservoirs to facilitate the loading of such agents, and to protect the agent from abrasion, extrusion, or other degradation during delivery and implantation.

Figure 1 illustrates an expandable medical device for directional delivery of one or more therapeutic agents 16. The openings 14 contain one or more therapeutic agents 16 for delivery to the lumen 116 of the blood vessel. In this case the therapeutic agent is thymosin β4, its analogues or derivatives. An optional cap 18 is shown in or can be adjacent the mural side of the openings. The cap 18 provides primarily luminal delivery of the thymosin β4, its analogues or derivatives. Alternately, the cap 18 can include and anti-restenotic agent.

A single opening 14 may contain more than one therapeutic agent or multiple openings may contain only one therapeutic agent. The therapeutic agent in each opening may be the same or different

The volume of therapeutic agent that can be delivered using openings 14 is about 3 to 10 times greater than the volume of a 5 micron coating covering a stent with the same stent/vessel wall coverage ratio. This much larger therapeutic agent capacity provides several advantages. The larger capacity can be used to deliver multi-drug combinations, each with independent release profiles, for improved efficacy. Also, larger capacity can be used to provide larger quantities of less aggressive drugs and to achieve clinical efficacy without the undesirable side-effects of more potent drugs, such as retarded healing of the endothelial layer.

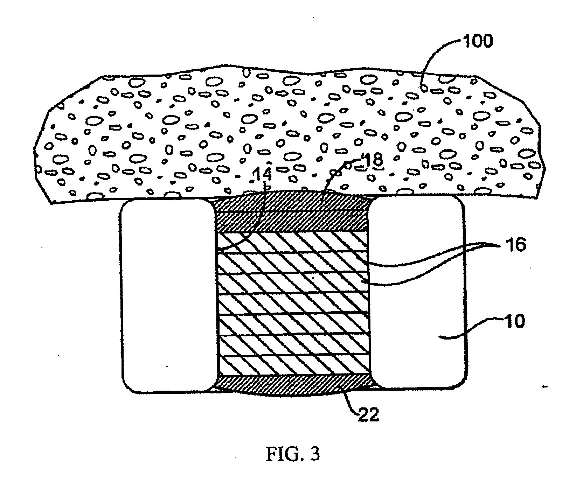

Figure 3 shows a cross section of a portion of a medical device 10 in which one or more therapeutic agents have been loaded into an opening 14 in multiple deposits. Although multiple discrete layers are shown for ease of illustration, the layers may be discrete layers with independent compositions or blended to form a continuous polymer matrix and agent inlay. For example, the layers can be deposited separately in layers of a drug, polymer, solvent composition which are then blended together in the openings by the action of the solvent. The agent may be distributed within an inlay uniformly or in a concentration gradient. Examples of some methods of creating such deposits and arrangements of layers are described in U.S. Patent Publication No. 2002/0082680, which is incorporated herein by reference in its entirety. The use of drugs in combination with polymers within the openings 14 allows the medical device 10 to be designed with drug release kinetics tailored to the specific drug delivery profile desired.

According to one embodiment, the openings have an area of at least 5x10⁻⁶ square inches, and preferably at least 10x10⁻⁶ square inches. In order to increase the overall volume of drug in the stent, the openings can be enlarged to about 15x10⁻⁶ square inches to about 50x10⁻⁶ square inches, with different holes having different cross sections. The volume of the holes can be 2x10⁻⁸ cubic inches to about 20x10⁻⁸ cubic inches.

In the example of Figure 3, the mural side of the openings are provided with a cap region 18 which is a region of polymer or other material having an erosion rate which is sufficiently slow to allow substantially all of the therapeutic agent in the therapeutic agent region 16 to be delivered from the luminal side of the opening prior to erosion of the cap region. The cap region 18 prevents loss of the therapeutic agent during transport, storage, and during the stent implantation procedure. However, the cap region 18 may be omitted where mural and luminal delivery of the agent is acceptable.

The base 22 can provide a seal during filling of the openings. The base 22 is preferably a rapidly degrading biocompatible material when providing luminal delivery.

Since the cap region 18 and therapeutic agent 16 are created independently, individual chemical compositions and pharmacokinetic properties can be imparted to each region. Numerous useful arrangements of such regions can be formed, some of which will be described below. Each of the regions may be formed from multiple deposits and include one or more agents in the same or different proportions from deposit to deposit. Changes in the agent concentration between deposits can be used to achieve a desired delivery or pharmacokinetic profile. For example, a decreasing release of drug for about 24 hours can be achieved. In another example, an initial burst followed by a constant release for about one week can be achieved. Substantially constant release rates over time period from a few hours to months can be achieved. The deposits may be solid, porous, or filled with other drugs or excipients.

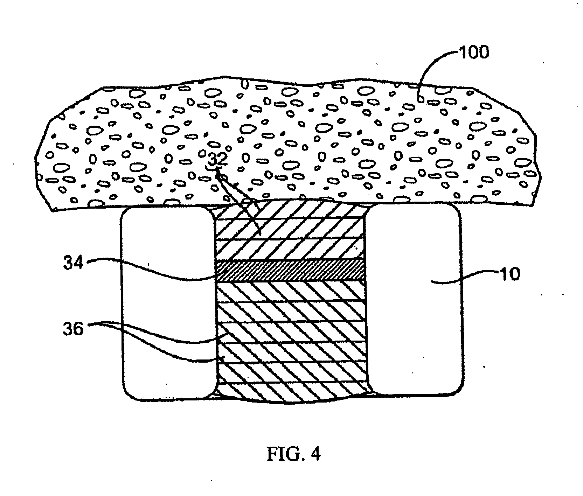

Figure 4 is a cross sectional view of a portion of an expandable medical device 10 including two or more therapeutic agents including an anti-ischemic agent and an anti-restenotic agent. Dual agent delivery systems such as that shown in Figure 4 can deliver two or more therapeutic agents in different directions for the treatment of different conditions or stages of conditions. For example, a dual agent delivery system may deliver a drug for treatment of ischemia 36 luminally and an anti-restenotic agent 32 murally from the same or different openings in the same drug delivery device.

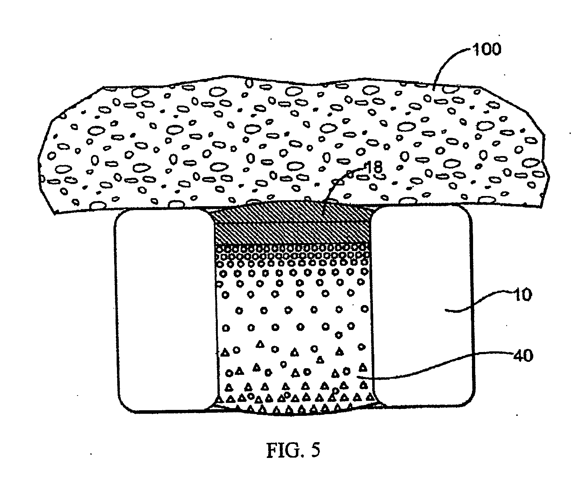

Figure 5 illustrates an expandable medical device 10 including an inlay 40 formed of a biocompatible matrix with first and second agents provided in the matrix for delivery according to different agent delivery profiles. As shown in Figure 5, a first drug illustrated by triangles (such as an anti-ischemic agent) is provided in the matrix with a concentration gradient such that the concentration of the drug is highest adjacent the luminal side of the opening and is lowest at the mural side of the opening. The second drug, illustrated by circles, is relatively concentrated in an area close to the mural cap region 18 in the opening. This configuration illustrated in Figure 5 results in delivery of two different agents with different delivery profiles and in different primary directions from the same inlay 40. In addition to, or as an alternative to the two agents provided in the matrix 40, one or more agents can be added to the cap region 18 or to a base region (not shown). For example, a drug sensitizer can be added to the base region of the embodiment of Figure 5.

In the embodiments described above, the therapeutic agent can be provided in the expandable medical device in a biocompatible matrix. The matrix can be bioresorbable or can be a permanent part of the device from which the therapeutic agent diffuses. One or more base regions, separating regions, and cap regions can be used to separate therapeutic agents within the openings or to prevent the therapeutic agents from degradation or delivery prior to implantation of the medical device.

In an exemplary embodiment, the stent is loaded with three regions, a base, a drug, and a cap. The base is a bioresorbable polymer, such as PLGA 50/50 or 85/15. Preferably, the base is formed of a fast degrading bioresorbable polymer. The base can also be formed of a non-bioresorbable polymer, such as one with pores for passage of the anti-ischemic agent, or a mixture of bioresorbable and non-bioresorbable polymers. The therapeutic agent, for example, thymosin β4, is provided in a bioresorbable polymer such as PLGA or a combination of polyvinyl pyrrolidone (PVP) and low molecular weight trehalose. The cap is one or more slow degrading polymers, such as PLA/PCL copolymer and/or PLGA 85/15.

### III. Drugs Incorporated into the Medical Devices For Improving Myocardial Function, Reducing Ischemic Injury and Restenosis

In one embodiment, a stent or other local delivery device may be used for local delivery of one or more anti-ischemic agents following acute myocardial infarction and reperfusion, or other damage to heart muscle. In preferred embodiments, the stent or another local delivery device is used for the delivery of an anti-ischemic agent which improves heart muscle function by promoting myocardial cell migration and proliferation, such as thymosin β4, its analogues or derivatives. One or more anti-restenotic drugs, such as pimecrolimus, sirolimus, everolimus, zotarolimus, biolimus, or paclitaxel, which reduces or inhibits restenosis may be delivered in combination with the ant-ischemic agent.

### A. Anti-Ischemic Agents

Thymosin β4 is a 43-amino-acid peptide that promotes myocardial and endothelial cell migration in the embryonic heart. An agent that promotes cell migration/penetration/proliferation in embryonic development of the heart can also decrease infact size in adults. In the adult heart, thymosin β4 also promotes cardiomyocyte migration and proliferation. In other organs, thymosin β4 promotes skin and corneal wound healing through similar effects on cell migration and angiogenesis.

The thymosin β4 or its analogues or derivatives can be delivered from the stent to stimulate native stem cells to migrate and differentiate in the heart to improve heart function. Alternatively, stem cells may be injected into the heart in combination with the delivery of thymosin β4.

In cell culture, including both embryonic and adult cardiomyocytes, exposure to thymosin β4 promotes skin and corneal wound healing through its effects on cell migration, angiogenesis and possibly cell survival enhanced survival when exposed to thymosin β4. The action of thymosin β4 is closely linked to the activation of the survival protein kinase Akt-1.

Thymosin β4 has numerous functions including sequestration of G-actin monomers and subsequent effects on actin-cytoskeletal organization necessary for cell motility, organogenesis and other cell biological events. B-thymosins can affect actin assembly based on their carboxyl-terminal affinity for actin.

*In vivo* studies in the animal laboratory models of acute ischemia and infarction induced by coronary occlusion-reperfusion show that thymosin β4 up regulates cardiac Akt-1 through phosphorylation, resulting in increased cardiomyocyte survival and increased cardiac functions, suggesting it may induce cardiomyocyte migration, survival and repair during acute coronary syndromes in man. The cells stimulated by thymosin β4 could be resident adult cardiac cells that dedifferentiate, stem cells from outside the heart, or resident cardiac stem cells that migrate and proliferate in the injured tissue.

Although the present application describes the use of thymosin β4 for reduction in ischemic injury following myocardial infarction, other related compounds which may be used as in the invention with or in place of thymosin β4 include thymosin β4 analoges, such as the family of β-thymosins (thymosin β4 isoforms), including thymosin β3, thymosin β4^{ala}, thymosin β9, thymosin β10, thymosin β11, thymosin β12, thymosin β13, thymosin β14, and thymosin β15, thymosin α1; pharmaceutically acceptable salts of thymosin; other peptides having G-actin sequestering, mobilizing, modulating, or binding properties; and agents that upregulate Akt-1, such as PI3 kinase activator.

In the treatmend of ischemic injury, the delivery of thymosin β4 to promote cell migration and proliferation can be used in combination with delivery of an agent which prevents reperfusion injury by inhibition of the initial inflammatory response following AMI. The delivery of thymosin β4 can also be combined with delivery of an agent which inhibits apoptotic cell death.

In scarless healing that occurs in the embryo, and in myocardial regeneration in certain species like newts and zebrafish, one of the most important factors is thymosin β4, which serves to up-regulate expression of growth factors (vascular endothelial growth factor, fibroblast growth factor, insulin-like growth factor-1, and hepatocyte growth factor and inhibit apoptotic factors (Bcl-2 and caspases). By creating an environment conducive to healing thymosin β4 alone, or in combination with prior intracoronary injection of an anti inflammatory or like substance, may also promote stem cell replication. Regeneration of myocardium requires the presence of uncommitted or partially committed cells that can differentiate into vascular and myocardial cells.

By infusiing thymosin β4 over a prolonged period of time, the drug delivery stent will establish a receptive environment for cardiac regeneration. A drug delivery stent including thymosin β4 can apply the mechanisms of embryonic scarless wound healing, cardiac regeneration in newts, and stem cell replication to myocardial repair and regeneration in humans. This strategy aims to recreate the essential features of the cellular environment that results in scarless healing. This strategy is ideally combined with one that stimulates the migration and proliferation of cardiac stem cells, or with one that stimulates transdifferentiation of cells adjacent to the injury area. The drug delivery stent delivers thymosin β4 during the critical period of myocardial healing following infarction, for restoration of myocardial function and for tissue regeneration.

Agents for the treatment of ischemic injury may also be delivered using a gene therapy-based approach in combination with an expandable medical device. Gene therapy refers to the delivery of exogenous genes to a cell or tissue, thereby causing target cells to express the exogenous gene product. Genes are typically delivered by either mechanical or vector-mediated methods. Mechanical methods include direct DNA microinjection, ballistic DNA-particle delivery, liposome-mediated transfection, and receptor-mediated gene transfer. Vector-mediated delivery typically involves recombinant virus genomes, including but not limited to those of retroviruses, adenoviruses, adeno-associated viruses, herpesviruses, vaccinia viruses, picornaviruses, alphaviruses, and papovaviruses.

Although the present application describes the delivery of thymosin β4 to reduce ischemia and reperfusion injury after AMI, the thymosin β4 stent can also treat heart muscle loss from other causes including myocarditis, chronic heart failure, or prior AMI.

### B. Anti-Restenotic Drugs

In another embodiment, one or more anti-restenotic drugs are delivered primarily from a mural side of a stent to inhibit restenosis, in addition to the agent or agents delivered primarily from the luminal side of the stent for reduction of ischemic injury. The primarily murally delivered agents may include antineoplastics, anti-mitotics, anti-inflammatories, antiangiogenics, angiogenic factors, anti-thrombotics, such as heparin, antiproliferatives, such as paclitaxel, sirolimus, everolimus, biolimus, zotarolimus, tacrolimus, and Pimecrolimus and derivatives thereof.

### C. Other Therapeutic Agents Incorporated into Medical Devices

Other therapeutically active, prophylactic or diagnostic agents can also be incorporated into the device, for delivery primarily murally, luminally, or bi-directionally. The primarily murally delivered agents may include antineoplastics, antimitotics, anti-inflammatories, anti-angiogenics, angiogenic factors, antirestenotics, anti-thrombotics such as heparin, antiproliferatives such as paclitaxel and rapamycin and derivatives thereof.

Other therapeutic agents for use with the present invention may, for example, take the form of small molecules, peptides, lipoproteins, polypeptides, polynucleotides encoding polypeptides, lipids, protein-drugs, protein conjugate drugs, enzymes, oligonucleotides and their derivatives, ribozymes, other genetic material, cells, antisense oligonucleotides, monoclonal antibodies, platelets, prions, viruses, bacteria, eukaryotic cells such as endothelial cells, stem cells, ACE inhibitors, monocyte/macrophages and vascular smooth muscle cells. Such agents can be used alone or in various combinations with one another. For instance, anti-inflammatories may be used in combination with antiproliferatives to mitigate the reaction of tissue to the antiproliferative. The therapeutic agent may also be a pro-drug, which metabolizes into the desired drug when administered to a host. In addition, therapeutic agents may be pre-formulated as microcapsules, microspheres, microbubbles, liposomes, niosomes, emulsions, dispersions or the like before they are incorporated into the matrix. Therapeutic agents may also be radioactive isotopes or agents activated by some other form of energy such as light or ultrasonic energy, or by other circulating molecules that can be systemically administered.

Exemplary classes of therapeutic agents include antiproliferatives, antithrombins (i.e., thrombolytics), immunosuppressants, antilipid agents, anti-inflammatory agents, antineoplastics including antimetabolites, antiplatelets, angiogenic agents, anti-angiogenic agents, vitamins, antimitotics, metalloproteinase inhibitors, NO donors, nitric oxide release stimulators, anti-sclerosing agents, vasoactive agents, endothelial growth factors, beta blockers, AZ blockers, hormones, statins, insulin growth factors, antioxidants, membrane stabilizing agents, calcium antagonists (i.e., calcium channel antagonists), retinoids, anti-macrophage substances, antilymphocytes, cyclooxygenase inhibitors, immunomodulatory agents, angiotensin converting enzyme (ACE) inhibitors, anti-leukocytes, high-density lipoproteins (HDL) and derivatives, cell sensitizers to insulin, prostaglandins and derivatives, anti-TNF compounds, hypertension drugs, protein kinases, antisense oligonucleotides, cardio protectants, petidose inhibitors (increase blycolitic metabolism), endothelin receptor agonists, interleukin-6 antagonists, anti-restenotics, vasodilators, and other miscellaneous compounds.

Antiproliferatives include, without limitation, paclitaxel, actinomycin D, rapamycin, everolimus, ABT-578, tacrolimus, cyclosporin, and pimecrolimus.

Antithrombins include, without limitation, heparin, aspirin, sulfinpyrazone, ticlopidine, ABCIXIMAB, eptifibatide, tirofiban HCL, coumarines, plasminogen, α₂-antiplasmin, streptokinase, urokinase, bivalirudin, tissue plasminogen activator (t-PA), hirudins, hirulogs, argatroban, hydroxychloroquin, BL-3459, pyridinolcarbamate, Angiomax, and dipyridamole.

Immunosuppressants include, without limitation, cyclosporine, sirolimus, tacrolimus, zotarolimus, everolimus, biolimus, etoposide, and mitoxantrone.

Antilipid agents include, without limitation, HMG CoA reductase inhibitors, nicotinic acid, probucol, and fibric acid derivatives (e.g., clofibrate, gemfibrozil, gemfibrozil, fenofibrate, ciprofibrate, and bezafibrate).

Anti-inflammatory agents include, without limitation, pimecrolimus, salicylic acid derivatives (e.g., aspirin, insulin, sodium salicylate, choline magnesium trisalicylate, salsalate, dflunisal, salicylsalicylic acid, sulfasalazine, and olsalazine), para-amino phenol derivatives (e.g., acetaminophen), indole and indene acetic acids (e.g., indomethacin, sulindac, and etodolac), heteroaryl acetic acids (e.g., tolmetin, diclofenac, and ketorolac), arylpropionic acids (e.g., ibuprofen, naproxen, flurbiprofen, ketoprofen, fenoprofen, and oxaprozin), anthranilic acids (e.g., mefenamic acid and meclofenamic acid), enolic acids (e.g., piroxicam, tenoxicam, phenylbutazone and oxyphenthatrazone), alkanones (e.g., nabumetone), glucocorticoids (e.g., dexamethaxone, prednisolone, and triamcinolone), pirfenidone, and tranilast.

Antineoplastics include, without limitation, nitrogen mustards (e.g., mechlorethamine, cyclophosphamide, ifosfamide, melphalan, and chlorambucil), methylnitrosoureas (e.g., streptozocin), 2-chloroethylnitrosoureas (e.g., carmustine, lomustine, semustine, and chlorozotocin), alkanesulfonic acids (e.g., busulfan), ethylenimines and methylmelamines (e.g., triethylenemelamine, thiotepa and altretamine), triazines (e.g., dacarbazine), folic acid analogs (e.g., methotrexate), pyrimidine analogs (5-fluorouracil, 5-fluorodeoxyuridine, 5-fluorodeoxyuridine monophosphate, cytosine arabinoside, 5-azacytidine, and 2',2'-difluorodeoxycytidine), purine analogs (e.g., mercaptopurine, thioguanine, azathioprine, adenosine, pentostatin, cladribine, and erythrohydroxynonyladenine), antimitotic drugs (e.g., vinblastine, vincristine, vindesiue, vinorelbine, paclitaxel, docetaxel, epipodophyllotoxins, dactinomycin, daunorubicin, doxorubicin, idarubicin, cpirubicin, mitoxantrone, bleomycins, plicamycin and mitomycin), phenoxodiol, etoposide, and platinum coordination complexes (e.g., cisplatin and carboplatin).

Antiplatelets include, without limitation, insulin, dipyridamole, tirofiban, eptifibatide, abciximab, and ticlopidine.

Angiogenic agents include, without limitation, phospholipids, ceramides, cerebrosides, neutral lipids, triglycerides, diglycerides, monoglycerides lecithin, sphingosides, angiotensin fragments, nicotine, pyruvate thiolesters, glycerol-pyruvate esters, dihydoxyacetone-pyruvate esters and monobutyrin.

Anti-angiogenic agents include, without limitation, endostatin, angiostati, fumagillin and ovalicin.

Vitamins include, without limitation, water-soluble vitamins (e.g., thiamin, nicotinic acid, pyridoxine, and ascorbic acid) and fat-soluble vitamins (e.g., retinal, retinoic acid, retinaldehyde, phytonadione, menaqinone, menadione, and alpha tocopherol).

Antimitotics include, without limitation, vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin.

Metalloproteinase inhibitors include, without limitation, TIMY-1, TIMP-2, TIMP-3, and SmaPI.

NO donors include, without limitation, L-arginine, amyl nitrite, glyceryl trinitrate, sodium nitroprusside, molsidomine, diazeniumdiolates, S-nitrosothiols, and mesoionic oxatriazole derivatives.

NO release stimulators include, without limitation, adenosine.

Mti-sclerosing agents include, without limitation, collagenases and halofuginone.

Vasoactive agents include, without limitation, nitric oxide, adenosine, nitroglycerine, sodium nitroprusside, hydralazine, phentolamine, methoxamine, metaraminol, ephedrine, trapadil, dipyridamole, vasoactive intestinal polypeptides (VIP), arginine, and vasopressin.

Endothelial growth factors include, without limitation, VEGF (Vascular Endothelial Growth Factor) including VEGF-121 and VEG-165, HGF (Fibroblast Growth Factor) including FGF-1 and FGF-2, HGF (Hepatocyte Growth Factor), and Ang1 (Angiopoietin 1).

Beta blockers include, without limitation, propranolol, nadolol, timolol, pindolol, labetalol, metoprolol, atenolol, esmolol, and acebutolol.

Hormones include, without limitation, progestin, insulin, the estrogens and estradiols (e.g., estradiol, estradiol valerate, estradiol cypionate, ethinyl estradiol, mestranol, quinestrol, estrond, estrone sulfate, and equilin).

Statins include, without limitation, mevastatin, lovastatin, simvastatin, pravastatin, atorvastatin, and fluvastatin.

Insulin growth factors include, without limitation, IGF-1 and IGF-2.

Antioxidants include, without limitation, vitamin A, carotenoids and vitamin E.

Membrane stabilizing agents include, without limitation, certain beta blockers such as propranolol, acebutolol, labetalol, oxprenolol, pindolol and alprenolol.

Calcium antagonists include, without limitation, amlodipine, bepridil, diltiazem, felodipine, isradipine, nicardipine, nifedipine, nimodipine and verapamil.

Retinoids include, without limitation, all-trans-retinol, all-trans-14-hydroxyretroretinol, all-trans-retinaldehyde, all-trans-retinoic acid, all-trans-3,4-didehydroretinoic acid, 9-cis-retinoic acid, 11-cis-retinal, 13-cis-retinal, and 13-cis-retinoic acid.

Anti-macrophage substances include, without limitation, NO donors.

Anti-leukocytes include, without limitation, 2-CdA, IL-1 inhibitors, anti-CD116/CD18 monoclonal antibodies, monoclonal antibodies to VCAM, monoclonal antibodies to ICAM, and zinc protoporphyrin.

Cyclooxygenase inhibitors include, without limitation, Cox-1 inhibitors and Cox-2 inhibitors (e.g., CELEBREX® and VIOXX®).

Immunomodulatory agents include, without limitation, immunosuppressants (see above) and immunostimulanls (e.g., levamisole, isoprinosine, Interferon alpha, and Interleukin-2).

ACE inhibitors include, without limitation, benazepril, captopril, enalapril, fosinopril sodium, lisinopril, quinapril, ramipril, spirapril, and 2B3 ACE inhibitors.

Cell sensitizers to insulin include, without limitation, glitazones, P PAR agonists and metformin.

Antisense oligonucleotides include, without limitation, resten-NG.

Cardio protectants include, without limitation, VIP, pituitary adenylate cyclase-activating peptide (PACAP), apoA-I milano, amlodipine, nicorandil, cilostaxone, and thienopyridine.

Petidose inhibitors include, without limitation, omnipatrilat.

Anti-restenotics include, without limitation, include vincristine, vinblastine, actinomycin, epothilone, paclitaxel, paclitaxel derivatives (e.g., docetaxel), sirolimus, rapamycin derivatives, everolimus, tacrolimus, zotarolimus, biolimus, and pimecrolimus.

PPAR gamma agonists include, without limitation, farglitizar, rosiglitazone, muraglitazar, pioglitazone, troglitazone, balaglitazone, and LBM-642.

Miscellaneous compounds include, without limitation, Adiponectin.

Agents may also be delivered using a gene therapy-based approach in combination with an expandable medical device. Gene therapy refers to the delivery of exogenous genes to a cell or tissue, thereby causing target cells to express the exogenous gene product. Genes are typically delivered by either mechanical or vector-mediated methods.

Some of the agents described herein may be combined with additives which preserve their activity. For example additives including surfactants, antacids, antioxidants, and detergents may be used to minimize denaturation and aggregation of a protein drug. Anionic, cationic, or nonionic detergents may be used. Examples of nonionic additives include but are not limited to sugars including sorbitol, sucrose, trehalose, pentaerythritol, glycine, mannitol; dextrans including dextran, carboxy methyl (CM) dextran, diethylamino ethyl (DEAE) dextran; sugar derivatives including D-glucosaminic acid, and D-glucose diethyl mercaptal; synthetic polyethers including polyethylene glycol (PEF and PEO) and polyvinyl pyrrolidone (PVP); carboxylic acids including D-lactic acid, glycolic acid, and propionic acid; detergents with affinity for hydrophobic interfaces including n-dodecyl-β-D-maltoside, n-octyl-β-D-glucoside, PEO-fatty acid esters (e.g. stearate (myrj 59) or oleate), PEO-sorbitan-fatty acid esters (e.g. Tween 80, PEO-20 sorbitan monooleate), sorbitan-fatty acid esters (e.g. SPAN 60, sorbitan monostearate), PEO-glyceryl-fatty acid esters; glyceryl fatty acid esters (e.g. glyceryl monostearate), PEO-hydrocarbon-ethers (e.g. PEO-10 oleyl ether; triton X-100; and Lubrol. Examples of ionic detergents include but are not limited to fatty acid salts including calcium stearate, magnesium stearate, and zinc stearate; phospholipids including lecithin and phosphatidyl choline; CM-PEG; cholic acid; sodium dodecyl sulfate (SDS); docusate (AOT); and taumocholic acid.

Agents for the treatment of ischemic injury may also be delivered using a gene therapy-based approach in combination with an expandable medical device. Gene therapy refers to the delivery of exogenous genes to a cell or tissue, thereby causing target cells to express the exogenous gene product Genes are typically delivered by either mechanical or vector-mediated methods. Mechanical methods include, but are not limited to, direct DNA microinjection, ballistic DNA-particle delivery, liposome-mediated transfection, and receptor-mediated gene transfer. Vector-mediated delivery typically involves recombinant virus genomes, including but not limited to those of retroviruses, adenoviruses, adeno-associated viruses, herpesviruses, vaccinia viruses, picornaviruses, alphaviruses, and papovaviruses.

### E. Additives

Therapeutic agents may be pre-formulated as microcapsules, microspheres, microbubbles, liposomes, niosomes, emulsions, or dispersions prior to incorporation into the delivery matrix.

Any of the pharmaceutically acceptable additives can be combined with the therapeutically active agents prior to or at the time of encapsulation. These may include surfactants, buffering agents, antioxidants, bulking agents, dispersants, pore forming agents, and other standard additives. Surfactants may be used to minimize denaturation and aggregation of a drug. Anionic, cationic, or nonionic surfactants may be used. Examples of nonionic surfactants include but are not limited to sugars including sorbitol, sucrose, trehalose; dextrans including dextran, carboxy methyl (CM) dextran, diethylamino ethyl (DEAE) dextran; polysaccharides including trehalose and mannitol; sugars and sugar derivatives including D-glucosaminic acid and D-glucose diethyl mercaptal; synthetic polyethers including polyethylene glycol (PEG) and polyvinyl pyrrolidone (PVP); carboxylic acids including D-lactic acid, glycolic acid, and propionic acid; detergents with affinity for hydrophobic interfaces including n-dodecyl-.beta.-D-maltoside, n-octyl-.beta.-D-glucoside, PEO-fatty acid esters (e.g. stearate (myrj 59) or oleate), PEO-sorbitan-fatty acid esters (e.g. Tween 80, PEO-20 sorbitan monooleate), sorbitan-fatty acid esters (e.g. SPAN 60, sorbitan monostearate), PEO-glyceryl-fatty acid esters; glyceryl fatty acid esters (e.g. glyceryl monostearate), PEO-hydrocarbon-ethers (e.g. PEO-10 oleyl ether; triton X-100; and Lubrol. Examples of ionic detergents include but are not limited to fatty acid salts including calcium stearate, magnesium stearate, and zinc stearate; phospholipids including lecithin and phosphatidyl choline; CM-PEG; cholic acid; sodium dodecyl sulfate (SDS); docusate (AOT); and taumocholic acid.

### IV. Methods of Treatment

### A. Method of Locally Delivering Drugs to Reduce Ischemic Injury

In one embodiment, one or more drugs which are suited for the reduction of ischemic injury are delivered at or near the site of a reopened occlusion following myocardial infarction or other acute ischemic syndromes. The delivery of the anti-ischemic agent at or near the site of the previous occlusion allows the drugs to be delivered by the blood flow downstream to the reperfused tissue. The drugs can be delivered by a stent containing drugs in openings in the stent as described above. The drugs can also be delivered by a drug coated stent, an implant, microspheres, a catheter, coils, or other local delivery means.

For example, microspheres, coils, liposomes, or other small drug carriers can be delivered locally at or near the site of a previous occlusion with a catheter or drug delivery stent. These small drug carriers are released and pass downstream into the myocardium where they may implant themselves delivering the drug directly to the ischemic tissue.

The anti-ischemic agent can be released over an administration period which is determined based on the mode of action of the drug delivered. For example, thymosin β4, its analogues, isoforms, and derivatives may be delivered over an administration period of from a hour up to two months. Preferably thymosin β4, its analogues, isoforms, and derivatives are delivered over a period of at least 1 day, more preferably at least 3 days, and more preferably about 3-30 days.

In one example, an anti-ischemic agent for reduction of ischemic injury is delivered from a stent primarily in a luminal direction with minimal drug being delivered directly from the stent in the direction of the vessel wall. This stent may be placed alone in the occlusion or may be placed in addition to another stent (bare stent or drug eluting delivery stent) placed in connection with an angioplasty procedure. The stent for delivery of anti-ischemic agent(s) may be placed within or adjacent another previously placed stent. The implantation site for the stent may be at or near the site of the occlusion. An implantation site may also be selected at or near a location of a plaque rupture site or a vessel narrowing. The stent may be a permanent or biodegradable stent.

In another example, the thymosin β4, its analogues, isoforms, or derivative can be delivered with a second anti-ischemic agent for treatment of ischemic injury may be delivered with the two agents delivered over different administration periods depending on the mode of action of the agents. For example, a fast acting agent may be delivered over a short period of a few minutes while a slower acting agent is delivered over several hours or days.

### B. Method of Locally Delivering Drugs to Reduce Ischemic Injury and Inhibit Restenosis

In preferred embodiments, an anti-restenotic agent is delivered primarily from a mural side of a stent to inhibit restenosis in addition to the anti-ischemic agent, which is delivered primarily from the luminal side of the stent. In one example, the anti-ischcmic agent is delivered at a first delivery rate for a first administration period, such as over a period of about 1 to about 30 days, while the anti-restenotic drug is delivered at a second delivery rate for a second administration period, such as over a period of about 30 days or longer.

Other primarily murally delivered agents include antineoplastics, antiangiogenics, anti-thrombotics, such as heparin, antiproliferatives, such as paclitaxel, sirolimus, pimecrolimus, everolimus, zotarolimus, biolimus and derivatives thereof.

### V. Pharmaceutically Acceptable Formulations

The compounds, or pharmaceutically acceptable salts thereof, including their polymorphic variations, can be formulated with pharmaceutically acceptable carriers. The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, solvent or an encapsulating material such as liposomes, polyethylene glycol (PEG), PEGylated liposomes, or particles, which is compatible with the other ingredients of the formulation and not injurious to the patient.

The phrases "systemic administration" and "administered systemically" as used herein mean the administration of a compound, drug or other material other than directly into the central nervous system, such that it enters the patient's vascular system.

Formulation of drugs is discussed in, for example. Hoover, John E., Remington's Pharmaceutical Sciences, Mack Publishing Co., Easton, Pennsylvania (1975), and Liberman, H.A. and Lachman, L., Eds., Pharmaceutical Dosage Forms, Marcel Decker, New York, N.Y. (1980).

The active compounds (or pharmaceutically acceptable salts thereof) may be administered per se or in the form of a pharmaceutical composition wherein the active compound(s) is in admixture or mixture with one or more pharmaceutically acceptable carriers, excipients or diluents. Pharmaceutical compositions may be formulated in conventional manner using one or more physiologically acceptable carriers comprising excipients and auxiliaries which facilitate processing of the active compounds into preparations which can be used pharmaceutically. Proper formulation is dependent upon the route of administration chosen.

Examples of suitable coating materials include, but are not limited to, cellulose polymers such as cellulose acetate phthalate, hydroxypropyl cellulose, hydroxypropyl methylcellulose, hydroxypropyl methylcellulose phthalate and hydroxypropyl methylcellulose acetate succinate; polyvinyl acetate phthalate, acrylic acid polymers and copolymers, and methacrylic resins that are commercially available under the trade name EUDRAGIT® (Roth Pharma, Westerstadt, Germany), zein, shellac, and polysaccharides.

Additionally, the coating material may contain conventional carriers such as plasticizers, pigments, colorants, glidants, stabilization agents, pore formers and surfactants.

Optional pharmaceutically acceptable excipients present in the drug-containing tablets, beads, granules, particles, or inlays include, but are not limited to, diluents, binders, lubricants, disintegrants, colorants, stabilizers, and surfactants.

Binders are used to impart cohesive qualities to a solid dosage formulation, and thus ensure that a tablet or bead or granule remains intact after the formation of the dosage forms. Suitable binder materials include, but are not limited to, starch, pregelatinized starch, gelatin, sugars (including sucrose, glucose, dextrose, lactose and sorbitol), polyethylene glycol, waxes, natural and synthetic gums such as acacia, tragacanth, sodium alginate, cellulose, including hydroxypropylmethylcellulose, hydroxypropylcellulose, ethylcellulose, and veegum, and synthetic polymers such as acrylic acid and methacrylic acid copolymers, methacrylic acid copolymers, methyl methacrylate copolymers, aminoalkyl methacrylate copolymers, polyacrylic acid/polymethacrylic acid and polyvinylpyrrolidone.

Disintegrants are used to facilitate dosage form disintegration or "breakup" after administration, and generally include, but are not limited to, starch, sodium starch glycolate, sodium carboxymethyl starch, sodium carboxymethylcellulose, hydroxypropyl cellulose, pregelatinized starch, clays, cellulose, alginine, gums or cross linked polymers, such as cross-linked PVP (Polyplasdone XL from GAF Chemical Corp).

Stabilizers are used to inhibit or retard drug decomposition reactions which include, by way of example, oxidative reactions.

Surfactants may be anionic, cationic, amphoteric or nonionic surface active agents. Suitable anionic surfactants include, but are not limited to, those containing carboxylate, sulfonate and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-1 50 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, Poloxamer^{®} 401, stearoyl monoisopropanolamide, and polyoxyethylene hydrogenated tallow amide. Examples of amphoteric surfactants include sodium N-dodecyl-.beta.-alanine, sodium N-lauryl-.beta.-iminodipropionate, myristoamphoacetate, lauryl betaine and lauryl sulfobetaine.

If desired, the dosage forms may also contain minor amount of nontoxic auxiliary substances such as wetting or emulsifying agents, dyes, pH buffering agents, or preservatives.

### VI. Exemplary Descriptions

### A. Thymosin β4 Stent

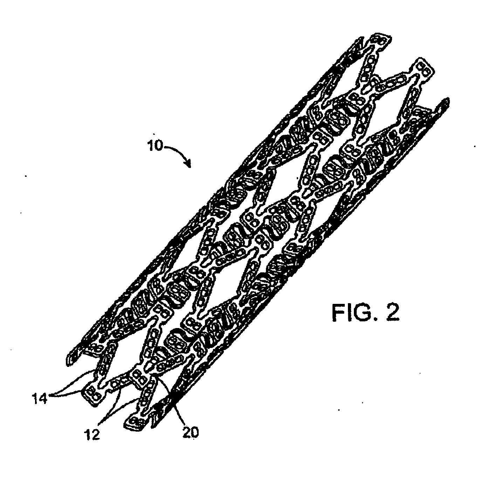

A drug delivery stent substantially equivalent to the stent illustrated in Figures 2 and 3 having an expanded size of about 3 mm x 30 mm is loaded with thymosin β4 with a total dosage of about 1 micrograms to 5 milligrams and with pimecrolimus with a total dosage of about 100-600 micrograms in the following manner. The stent is positioned on a mandrel and an optional quick degrading base is deposited into the openings in the stent. The quick degrading base is PLGA. A plurality of deposits of thymosin β4 and low molecular weight PLGA are then deposited into the openings to form an inlay of drug for the reduction of ischemic injury.

The compositions are deposited in a dropwise manner and are delivered in liquid form by use of a suitable organic solvent, such as DMSO, NMP, or DMAc. The thymosin β4 and polymer matrix are combined and deposited in a manner to achieve a thymosin β4 delivery profile which results in essentially 100% released in about 3 to 14 days.

The thymosin β4 dosage provided on the stent described is about 10 micrograms to about 5 milligrams. A plurality of deposits of high molecular weight PLGA, or other slow degrading polymer, and pimecrolimus are deposited over the thymosin β4 to provide a cap which delivers pimecrolimus from the cap to the mural side of the stent and the vessel walls. The resorbtion rate of the pimecrolimus cap is selected to deliver pimecrolimus continuously over an administration period of about 4 to about 15 weeks.

In another example, the thymosin β4 stent described above is modified by providing the thymosin β4 in a polymer matrix including combination of polyvinyl pyrrolidone (PVP) and low molecular weight trehalose. In addition, one or more stabilization agents can be added to the thymosin β4 and/or the pimecrolimus to increase shelf life. For example, BHT can be used.

It is understood that the disclosed methods are not limited to the particular methodology, protocols, and reagents described as these may vary. It is also to be understood that the terminology used herein is for the purpose of describing particular embodiments only, and is not intended to limit the scope of the present invention which will be limited only by the appended claims.

Certain embodiments of the invention are set out in the following numbered clauses.
1. A method for reducing tissue damage following ischemic injury in a patient, the method comprising:
   administering to the patient thymosin β4 or an isoform thereof, wherein the thymosin β4 or isoform thereof is administered locally to or near the site of ischemic injury at a dosage which reduces tissue damage due to ischemia,
2. The method of clause 1, wherein the thymosin β4 or isoform thereof is administered in a medical device implanted at or near the site of ischemic injury.
3. The method of clause 2, wherein the device is selected from the group consisting of stents, polymeric delivery devices, polymeric particles and polymeric coatings.
4. The method of clause 3, wherein the thymosin β4 or isoform thereof is administered into a blood vessel.
5. The method of clause 2, wherein an anti-restenotic drug is delivered primarily from a mural side of the medical device, and wherein the thymosin β4 or isoform thereof is delivered primarily from a luminal side of the medical device.
6. The method of clause 2, wherein the medical device is a stent.
7. The method of clause 1, wherein the thymosin β4 or isoform thereof is thymosin β4.
8. The method of clause 5, wherein the anti-restenotic agent is selected from the group of compounds consisting of antineoplastics, antimitotics, antiangiogenics, angiogenic factors, anti-thrombotics, antiproliferatives, and anti-inflammatories.
9. The method of clause 5, wherein the anti-restenotic agent is pimecrolimus, sirolimus or paclitaxel.
10. The method of clause 1, wherein the thymosin β4 or isoform thereof is delivered from a polymer.
11. The method of clause 10, wherein the polymer is in the form of polymeric coatings or particles located at or near an occlusion site.
12. The method of clause 1, wherein the thymosin β4 or isoform thereof and a biocompatible polymer matrix are deposited within openings in an implantable medical device for local delivery to an occlusion site.
13. The method of clause 12, wherein the thymosin β4 or isoform thereof and a biocompatible polymer are deposited within openings in an implantable medical device and wherein a cap region is provided which substantially prevents delivery of the thymosin β4 or isoform thereof to the artery wall.
14. A method for improving heart muscle function in a patient, the method comprising:
   administering to the patient thymosin β4 or an isoform thereof from a stent at a dosage which promotes myocardial cell migration and proliferation.
15. The method of clause 14, wherein the thymosin β4 or isoform thereof is administered the stent implanted at or near a site of a previous myocardial infarction.
16. The method of clause 14, wherein an anti-restenotic drug is delivered primarily from a mural side of the stent, and wherein the thymosin β4 or isoform thereof is delivered primarily from a luminal side of the stent.
17. The method of clause 14, wherein the thymosin β4 or isoform thereof is thymosin β4.
18. The method of clause 16, wherein the anti-restenotic agent is selected from the group of compounds consisting of antineoplastics, antimitotics, antiangiogenics, angiogenic factors, anti-thrombotics, antiproliferatives, and anti-inflammatories.
19. The method of clause 16, wherein the anti-restenotic agent is pimecrolimus, sirolimus or paclitaxel.
20. The method of clause 14, wherein the thymosin β4 or isoform thereof is affixed on the stent in a polymer.
21. The method of clause 14, wherein the thymosin β4 or isoform thereof and a biocompatible polymer matrix are deposited within openings in the stent for local delivery.
22. An implantable stent for reducing tissue damage following ischemic injury in a patient, comprising:
   an expandable stent structure:
23. The stent of clause 22, wherein the thymosin β4 or isoform thereof is provided on the stent at a therapeutic dosage of about 10 micrograms to about 1 milligram.
24. The stent of clause 22 or 23, wherein the thymosin β4 or isoform thereof is affixed to the stent by depositing in holes in the stent. Embodiments where the thymosin β4 or isoform thereof is affixed to the stent by depositing in holes in the stent are preferred.

## Claims

1. Thymosin β4 or an isoform thereof for use in a method for reducing tissue damage following ischemic injury in a patient, the method comprising:
administering to the patient thymosin β4 or an isoform thereof, wherein the thymosin β4 or isoform thereof is administered locally to or near the site of ischemic injury at a dosage which reduces tissue damage due to ischemia.

2. Thymosin β4 or an isoform thereof according to claim 1, wherein the thymosin β4 or isoform thereof is administered in a medical device implanted at or near the site of ischemic injury.

3. Thymosin β4 or an isoform thereof according to claim 2, wherein the device is selected from the group consisting of stents, polymeric delivery devices, polymeric particles and polymeric coatings.

4. Thymosin β4 or an isoform thereof according to any of the preceding claims, wherein the thymosin β4 or isoform thereof is administered into a blood vessel.

5. Thymosin β4 or an isoform thereof according to claim 2 or claim 3, wherein an anti-restenotic drug is delivered primarily from a mural side of the medical device, and wherein the thymosin β4 or isoform thereof is delivered primarily from a luminal side of the medical device.

6. Thymosin β4 or an isoform thereof according to claim 2, claim 3 or claim 5, wherein the medical device is a stent.

7. Thymosin β4 or an isoform thereof according to any of the preceding claims, wherein the thymosin β4 or isoform thereof is thymosin β4.

8. Thymosin β4 or an isoform thereof according to claim 5, wherein the anti-restenotic agent is selected from the group of compounds consisting of antineoplastics, antimitotics, antiangiogenics, angiogenic factors, anti-thrombotics, antiproliferatives, and anti-inflammatories.

9. Thymosin β4 or an isoform thereof according to claim 5, wherein the anti-restenotic agent is pimecrolimus, sirolimus or paclitaxel.

10. Thymosin β4 or an isoform thereof according to any of the preceding claims, wherein the thymosin β4 or isoform thereof is delivered from a polymer.

11. Thymosin β4 or an isoform thereof according to claim 10, wherein the polymer is in the form of polymeric coatings or particles located at or near an occlusion site.

12. Thymosin β4 or an isoform thereof according to any of the preceding claims, wherein the thymosin β4 or isoform thereof and a biocompatible polymer matrix are deposited within openings in an implantable medical device for local delivery to an occlusion site.

13. Thymosin β4 or an isoform thereof according to claim 12, wherein the thymosin β4 or isoform thereof and a biocompatible polymer are deposited within openings in an implantable medical device and wherein a cap region is provided which substantially prevents delivery of the thymosin β4 or isoform thereof to the artery wall.

14. An implantable stent for reducing tissue damage following ischemic injury in a patient, comprising:
an expandable stent structure:
thymosin β4 or an isoform thereof affixed to the stent structure, wherein the thymosin β4 or isoform thereof reduces tissue damage due to ischemia.

15. An implantable stent for reducing tissue damage following ischemic injury in a patient, comprising:
an expandable stent structure:
an anti-ischemic agent which mitigates apoptosis following myocardial infarction, the anti-ischemic agent affixed to the stent structure and arranged for luminal delivery; and
an anti-restenotic agent affixed to the stent and arranged for mural delivery.

16. The stent of claim 15, wherein the anti-ischemic agent is thymosin β4 or an isoform thereof.

17. The stent of claims 15 or 16, wherein the anti-restenotic agent is pimecrolimus or sirolimus.

18. The stent of any of claims 15 to 17, wherein the anti-ischemic agent and the anti-restenotic agent are affixed in openings in the stent structure.

19. Thymosin β4 or an isoform thereof for use in a method for improving heart muscle function in a patient, the method comprising:
administering to the patient the thymosin β4 or isoform thereof from a stent at a dosage which promotes myocardial cell migration and proliferation.

20. Thymosin β4 or an isoform thereof according to claim 19, wherein the thymosin β4 or isoform thereof is administered from a stent implanted at or near a site of a previous mycordial infarction.

21. Thymosin β4 or an isoform thereof according to claim 19 or 20, wherein an anti-restenotic drug is delivered primarily from a mural side of the stent, and wherein the thymosin β4 or isoform thereof is delivered primarily from a luminal side of the stent.

22. Thymosin β4 or an isoform thereof according to any one of claims 19 to 21, wherein the thymosin β4 or isoform thereof is thymosin β4.

23. Thymosin β4 or an isoform thereof according to claim 21, wherein the anti-restenotic agent is selected from the group of compounds consisting of antineoplastics, antimitotics, antiangiogenics, angiogenic factors, anti-thrombotics, antiproliferatives, and anti-inflammatories.

24. Thymosin β4 or an isoform thereof according to claim 21, wherein the anti-restenotic agent is pimecrolimus, sirolimus or paclitaxel.

25. Thymosin β4 or an isoform thereof according to any one of claims 19 to 24, wherein the thymosin β4 or isoform thereof is affixed on the stent in a polymer.

26. Thymosin β4 or an isoform thereof according to any one of claims 19 to 25, wherein the thymosin β4 or isoform thereof and a biocompatible polymer matrix are deposited within openings in the stent for local delivery.

27. An implantable stent for improving heart muscle function in a patient, comprising:
an expandable stent structure:
thymosin β4 or an isoform thereof affixed to the stent structure.

28. The stent of claim 14 or 27, wherein the thymosin β4 or isoform thereof is affixed to the stent in a polymer formulation adapted to release the thymosin β4 or isoform thereof over an administration period of at least one hour.

29. The stent of claim 14 or 27, wherein the thymosin β4 or isoform thereof is affixed to the stent in a polymer formulation adapted to release the thymosin β4 or isoform thereof over an administration period of about 1 to about 30 days.

30. The stent of any one of claims 14 and 27 to 29, wherein the thymosin β4 or isoform thereof is provided on the stent at a therapeutic dosage of about 10 micrograms to about 1 milligram.

31. The stent of any one of claims 14 and 27 to 30, wherein the thymosin β4 or isoform thereof is deposited in holes in the stent.

32. The stent of any one of claims 14 and 27 to 31, further comprising an anti-restenotic agent affixed to the stent.

33. The stent of claim 32, wherein the anti-restenotic agent is affixed to the stent by depositing in holes in the stent.

34. The stent of claim 32 or 33, wherein the anti-restenotic agent is pimecrolimus, sirolimus, or paclitaxel.

35. Thymosin β4 or an isoform thereof for use in surgery.

36. Use of thymosin β4 or an isoform thereof in the manufacture of a medical device for improving heart muscle function or for reducing tissue damage following ischemic injury, wherein the medical device is used simultaneously, separately or sequentially with an anti-restenotic drug.

37. Use of an anti-restenotic drug in the manufacture of a medical device for improving heart muscle function or for reducing tissue damage following ischemic injury, wherein the medical device is used simultaneously, separately or sequentially with thymosin β4 or an isoform thereof.

38. Use of thymosin β4 or an isoform thereof and an anti-restenotic drug in the manufacture of a medical device for improving heart muscle function or for reducing tissue damage following ischemic injury.
